(19) Europäisches Patentamt

European Patent Office

Office européen des brevets

(11) Veröffentlichungsnummer: **0 306 744 B1**

(12) **EUROPÄISCHE PATENTSCHRIFT**

(45) Veröffentlichungstag der Patentschrift: **29.04.92**

(21) Anmeldenummer: **88113468.8**

(22) Anmeldetag: **19.08.88**

(51) Int. Cl.⁵: **A61F 2/38**

(54) **Tibiateil einer Kniegelenk-Endoprothese.**

(30) Priorität: **09.09.87 DE 3730175**

(43) Veröffentlichungstag der Anmeldung:
**15.03.89 Patentblatt 89/11**

(45) Bekanntmachung des Hinweises auf die
Patenterteilung:
**29.04.92 Patentblatt 92/18**

(84) Benannte Vertragsstaaten:
**AT BE CH DE ES FR GB GR IT LI LU NL SE**

(56) Entgegenhaltungen:
**EP-A- 0 177 776**
**US-A- 4 353 136**

(73) Patentinhaber: **S + G IMPLANTS GMBH**
**Grapengiesserstrasse 34**
**W-2400 Lübeck(DE)**

(72) Erfinder: **Grundei, Hans**
**Hamburger Strasse 89**
**W-2400 Lübeck(DE)**

(74) Vertreter: **Eisenführ, Speiser & Strasse**
**Martinistrasse 24**
**W-2800 Bremen 1(DE)**

## Beschreibung

Die Erfindung geht aus von einem Tibiateil einer Kniegelenk-Endoprothese nach dem Oberbegriff des Anspruches 1. Derartige Tibiateile sind z.B. durch die EP-A-0 177 776 bekannt.

Bei den vorerwähnten bekannten Tibiateilen ist es möglich, die Tibiaplateaus aus einem geeigneten Kunststoff nach einem Verschleiß auszutauschen, während die vorn überbrückten metallischen Schalen im Schienbein verankert bleiben. Es hat sich herausgestellt, daß dieser Plateauaustausch verhältnismäßig schwierig durchzuführen ist, da die Plateaus von den Seitenwandungen der metallischen Schalen eingefaßt sind und daher ein weites Auseinanderspreizen der beiden Gelenkteile eines Patienten erfordern, um die verschlissenen Plateaus herauszunehmen und die neuen Plateaus wieder einsetzen zu können.

Die Aufgabe der Erfindung besteht darin, bei Tibiateilen der eingangs erwähnten Art den Austausch verschlissener durch neue Plateaus wesentlich zu erleichtern, dabei aber eine einwandfreie Fixierung der Plateaus in den metallischen Schalen zu gewährleisten.

Diese Aufgabe wird nach der Erfindung durch die kennzeichnenden Merkmale des Anspruches 1 gelöst.

Durch diese Lösung ist es möglich, die Plateaus von vorn in die Schalen einzuschieben, wobei der Vorsprung der beiden parallelen Wandungen in die entsprechenden Ausnehmungen der Plateaus in bekannter Weise einfassen. Dabei wird die durch die Höhenverringerung der vorderen Schalenwandungen verschlechterte Fixierung der Plateaus dadurch wieder ausgeglichen und verbessert, daß die Klemme vorgesehen ist, die sich beim Aufsetzen auf die Brücke der beiden Schalen mit der Brücke lösbar verbindet und mit den quergerichteten Zapfen in obere seitliche Ausnehmungen der Plateaus eingreift und die Plateaus damit in ihrer Lage in den Schalen sichert.

Vorteilhaft besteht die Klemme aus einem rechtwinkligen Blech, dessen einer Schenkel sich oben auf der Brücke abstützt und das seitlich mit nach außen gerichteten Zapfen versehene Arme aufweist und dessen anderer Schenkel mit einem klemmenden Einschnitt über einen in den Freiraum zwischen den beiden Schalen gerichteten Zapfen der Brücke greift.

Die Erfindung wird nachstehend anhand der Zeichnung erläutert. Es zeigt:

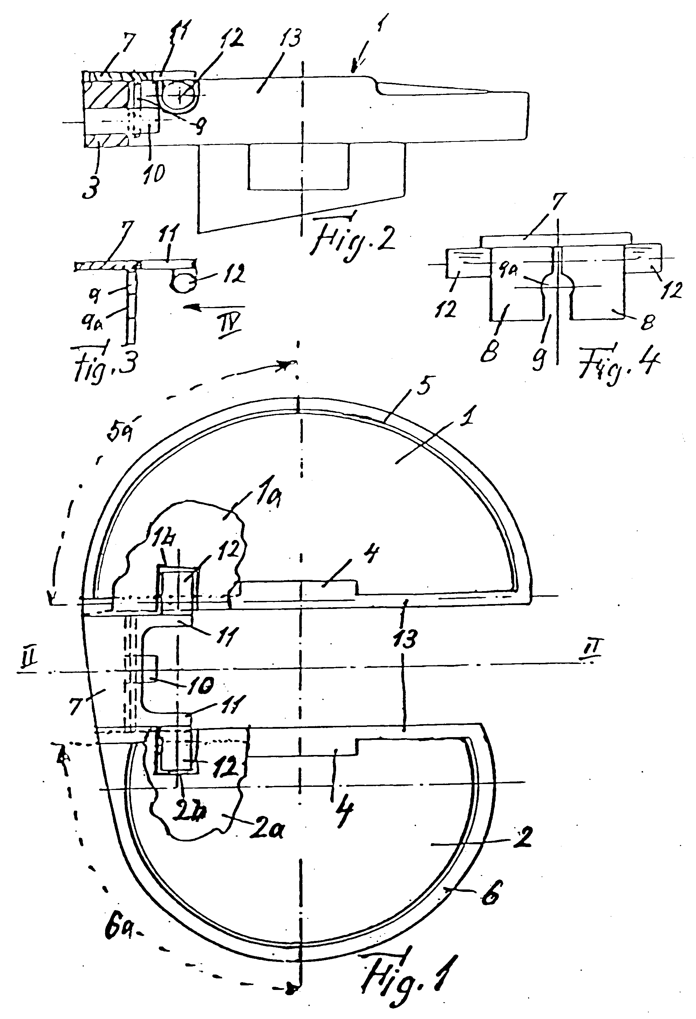

Figur 1     eine Aufsicht auf den Tibiateil einer Kniegelenk-Endoprothese mit der Klemme nach der Erfindung,

Figur       einen Schnitt nach der Linie II-II der Figur 1

Figur 3     die Klemme nach Figur 2, getrennt von dem Tibiateil dargestellt,

Figur 4     eine Ansicht der Klemme in Richtung des Pfeiles IV der Figur 3.

Das Tibiateil der Kniegelenk-Endoprothese besteht aus den beiden metallischen Schalen 1 und 2, die vorn durch eine Brücke 3 verbunden sind. In die Schalen 1,2 werden Tibiaplateaus 1a, 2a aus Kunststoff eingesetzt, auf denen die Kufen eines Femurteiles gleiten, wobei ein Fixiervorsprung 4 der Schalenwandungen 13 in eine entsprechende zugekehrte Ausnehmung der Plateaus 1a,2a eingreift. Die Tibiaplateaus 1a,2a sind in Fig.1 nur zum Teil dargestellt.

Gemäß der Erfindung ist die Seitenwand 5,6 der beiden Schalen 1, 2 im vorderen gebogenen Teil auf der Länge 5a, 6a in der Höhe gegenüber der sonstigen Seitenwand herabgesetzt, so daß dann die Plateaus 1a, 2a von vorn in die Schalen eingeschoben und verschlissene Plateaus umgekehrt aus den Schalen herausgenommen werden können.

Durch die herabgesetzte Höhe der Seitenwandungen 5a,6a wird die Fixierung der Plateaus 1a, 2a in den Schalen etwas ungünstig beeinflußt. Um dies auszugleichen, kommt eine Fixierklemme zur Anwendung. Diese Klemme besteht aus einem rechtwinkligen Blech, dessen einer Schenkel 7 sich auf der Oberfläche der Brücke 3 abstützt und dessen anderer Schenkel 8 mit einem mittigen aufrechten Einschnitt 9 versehen ist und mit diesem Einschnitt 9 über einen in den Freiraum zwischen den beiden Schalen gerichteten Zapfen 10 der Brücke 3 gedrückt wird und dabei die beiden Flächenhälften des Schenkels 8 auseinanderdrückt, bis der Zapfen 10 in die Erweiterung 9a des Einschnittes 9 eingreift bzw. einschnappt. Damit ist die Klemme fest und lösbar mit der Brücke verbunden.

Der obere Schenkel 7 der Klemme besitzt seitlich je einen Arm 11, an deren Enden je ein Fixierzapfen 12 befestigt ist, der in den Bereich der Schalen 1,2 hineinragt. Dabei sind die parallelen Schalenseitenwandungen 13 und die Tibiaplateaus 1a, 2a im Bereich der Zapfen 12 mit oberen Einschnitten 14, 24 versehen, und entsprechend sind die beiden in die Schalen 1,2 einzuschiebenden Tibiaplateaus 1a, 2a mit oberen Ausnehmungen 1b 2b versehen, in die sich die Zapfen 12 einlegen und dadurch die beiden Plateaus 1a, 2a in ihren Schalen 1,2 fixieren.

## Patentansprüche

1. Tibiateil einer Kniegelenk-Endoprothese, bei dem aus Kunststoff bestehende Tibiaplateaus (1a,2a) mit Gleitflächen für die Kufen des Femurteils in zwei vorn überbrückte Schalen (1,2) eingreifen, wobei Vorsprünge (4) der Schalen in Ausnehmungen der Tibiaplateaus eingreifen, dadurch gekennzeichnet, daß die nach vorn gerichteten bogenförmigen Seitenwände (5a,6a) der beiden Schalen (1,2) bis etwa zur größten Schalenbreite im Vergleich zum übrigen Teil der Seitenwände (5,6) eine geringere Höhe haben und daß das Tibiateil eine auf der vorderen Brücke (3) der Schalen aufsetzbare und fixierbare Klemme mit quergerichteten Zapfen (12) aufweist, welche in obere Ausnehmungen (1b,2b) der beiden Tibiaplateaus (1,2) eingreifen.

2. Tibiateil nach anspruch 1, dadurch gekennzeichnet, daß die Klemme aus einem rechtwinkligen Blech besteht, dessen einer Schenkel (7) sich oben auf der Brücke (3) abstützt und seitlich mit nach außen gerichteten Zapfen (12) versehen ist, und dessen anderer Schenkel (8) mit einem klemmenden Einschnitt (9) über einen in den Freiraum zwischen den beiden Schalen (1,2) gerichteten Zapfen (10) der Brücke (3) greift.

## Claims

1. A tibia component of a knee joint endoprosthesis, in which tibia plates (1a, 2a) made from plastic with gliding planes for the runners of the femur component engage in two shells (1, 2) which are bridged at the front, with projections (4) of the shells engaging in recesses of the tibia plates,
**characterised in that** the forwardly directed, curved side walls (5a, 6a) of the two shells (1, 2) have a smaller height in comparison with the remaining part of the side walls up to roughly the greatest shell width
**and in that** the tibia component comprises a clamp, which can be placed on and fixed to the front bridge (3) of the shells, with transverse bolt pins (12), which engage in upper recesses (1b, 2b) of the two tibia plates (1, 2).

2. A tibia component according to Claim 1,
**characterised in that** the clamp consists of a rectangular sheet, one arm (7) of which is supported at the top on the bridge (3) and is laterally provided with outwardly directed bolt pins (12), and the other arm (8) of which engages with a clamping notch (9) via a bolt pin (10) of the bridge (3) directed into the space between the two shells (1, 2).

## Revendications

1. Partie de tibia d'une endoprothèse pour l'articulation du genou, dans laquelle des plateaux de tibia (1a, 2a) constitués de matière synthétique et présentant des surfaces de glissement pour les patins de la partie de fémur pénètrent dans deux coques (1, 2) réunies par un pont à l'avant, des saillies (4) des coques pénétrant dans des évidements des plateaux de tibia, caractérisée en ce que les parois latérales (5a, 6a) arquées orientées vers l'avant des deux coques (1, 2) ont, jusqu'à approximativement la plus grande largeur de coque, une plus faible hauteur que la partie restante des parois latérales (5, 6) et en ce que la partie de tibia présente une pince qui peut être montée et fixée sur le pont avant (3) des coques et qui présente des tourillons (12) dirigés transversalement qui pénètrent dans des évidements supérieurs (1b, 2b) des deux plateaux de tibia (1, 2).

2. Partie de tibia suivant la revendication 1, caractérisée en ce que la pince est constituée d'une plaque en angle droit dont une aile (7) s'appuie en haut sur le pont (3) et est pourvue latéralement des tourillons (12) dirigés vers l'extérieur et dont l'autre aile (8) prend par une entaille (9) bloquante par-dessus un tourillon (10) du pont (3) qui est dirigé dans l'espace libre entre les deux coques (1, 2).

Fig.2

Fig.3

Fig.4

Fig.1